(19) Europäisches Patentamt
European Patent Office
Office européen des brevets

(11) Veröffentlichungsnummer: **0 532 566 B1**

# (12) EUROPÄISCHE PATENTSCHRIFT

(45) Veröffentlichungstag der Patentschrift: **08.03.95**

(51) Int. Cl.[6]: **C07F 13/00**, A61K 51/00

(21) Anmeldenummer: **91910279.8**

(22) Anmeldetag: **31.05.91**

(86) Internationale Anmeldenummer:
**PCT/DE91/00470**

(87) Internationale Veröffentlichungsnummer:
**WO 91/18908 (12.12.91 91/28)**

## (54) 99m-Tc-CYCLOPENTADIENYLCARBONYL -KOMPLEXE, VERFAHREN ZU IHRER HERSTELLUNG SOWIE IHRE VERWENDUNG IN DER DIAGNOSTIK.

(30) Priorität: **01.06.90 DE 4018172**
**08.09.90 DE 4028867**

(43) Veröffentlichungstag der Anmeldung:
**24.03.93 Patentblatt 93/12**

(45) Bekanntmachung des Hinweises auf die Patenterteilung:
**08.03.95 Patentblatt 95/10**

(84) Benannte Vertragsstaaten:
**AT BE CH DE DK ES FR GB GR IT LI LU NL SE**

(56) Entgegenhaltungen:
**EP-A- 0 203 815**

**Inorganic Chemistry, vol. 15, No. 11, November 1976, I G De Jong et al.:"Radiochemistry of organomolybdenum compounds. 2 formation of technetium compounds by beta-decay in etacyclopentadienyltricarbonylmolybdenum" pages 2588-2591**

**Chemical Abstracts, vol. 60, No. 7, 30 March 1964 (Columbus, Ohio, US) E O Fisher et al.:"Aromatic complexes of metals. LXXIII. Acylation of cyclopentadienylrhenium tricarbonyl and cyclopentadienyltevhnetiumtricarbonyl".**

(73) Patentinhaber: **INSTITUT FÜR DIAGNOSTIKFORSCHUNG GmbH AN DER FREIEN UNIVERSITÄT BERLIN**
**Spandauer Damm 130**
**D-14050 Berlin (DE)**

(72) Erfinder: **WENZEL, Martin**
**Bergengruenstr. 46**
**D-1000 Berlin 38 (DE)**
Erfinder: **SCHULZE, Paul-Eberhardt**
**Endestr. 10**
**D-1000 Berlin 39 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 532 566 B1

Rank Xerox (UK) Business Services
(3.10/3.09/3.3.3)

## Beschreibung

Die Erfindung betrifft den in den Patentansprüchen gekennzeichneten Gegenstand, d.h. Cyclopentadienylcarbonyl-$^{99m}$Tc-Komplexe, diese Komplexe enthaltende Mittel, ihre Verwendung als Diagnostika sowie Verfahren zur Herstellung dieser Komplexe und Mittel.

Seit längerer Zeit werden radioaktive Metallionen, meist gebunden an einen Komplexbildner, für die in vivo-Diagnostik verwendet. Hiervon ist Technetium-99m ($^{99m}$Tc) aufgrund seiner für diesen Zweck nahezu idealen physikalischen Eigenschaften - gute Absorption der Strahlung in entsprechenden Detektionsgeräten (Gammakamera, SPECT-Geräte) gegenüber einer geringeren Absorption im menschlichen Organismus und leichte Verfügbarkeit über einen Molybdän/Technetium-Generator - das am häufigsten in der klinischen Nuclearmedizin verwendete Radionuclid. Seine kurze Halbwertszeit von 6,02 h garantiert eine nur geringe Belastung des Patienten mit Gammastrahlung, zumal auch das Tochternuclid Technetium-99 nur eine vernachlässigbare Reststrahlung besitzt. Ein Nachteil des Technetiums ist allerdings seine komplizierte und noch nicht vollständig bekannte Komplex-Chemie. Technetium kann in einer Reihe von Oxidationsstufen (+7 bis -1) vorliegen, die die pharmakologischen Eigenschaften durch Veränderung der Ladung eines Komplexes stark verändern können. Es ist deswegen nötig, Komplexe zu synthetisieren, die das Technetium in einer definierten Oxidationsstufe binden und Redox-Reaktionen, die zu einer Redistribution des Pharmakons führen könnten, verhindern.Eine Reihe solcher $^{99m}$Tc-Komplexbildner sind bereits bekannt und werden klinisch angewendet. Bei neutralen Komplexen handelt es sich vielfach um Systeme, in denen das $^{99m}$Tc zwischen 2-4 Stickstoffatomen und 0-2 Schwefelatomen gebunden wird ($N_2S_2$-, $N_3S$-, Propylenaminoxim- und Isonitril-Komplexe). Vielfach ist jedoch die ungenügende Stabilität dieser $^{99m}$Tc-Komplexe ein wesentlicher Nachteil (Hung, J.C. et al.; J. Nucl. Med. 29: 1568 [1988]). In der Klinischen Anwendung muß deswegen z.B. HM-PAO (Hexamethyl-propylenaminoxim) innerhalb von 30 Minuten nach seiner Markierung mit $^{99m}$Tc-Pertechnetat appliziert werden, damit die Menge an Nebenprodukten, die die diagnostische Aussagekraft vermindern, nicht zu hoch wird.

Auch andere bisher bekannte Ansätze zur Kopplung von Chelatbildnern an sich selektiv anreichernde Substanzen können bisher als nicht zufriedenstellend betrachtet werden. Werden Werden funktionelle Gruppen des Komplexbildners zur Bindung an ein beliebiges niedermolekulares oder polymeres Molekül benutzt, so kommt es häufig zu einer Abschwächung der Komplexstabilität, d.h. ein diagnostisch nicht tolerierbarer Anteil des Radio-Isotops wird aus dem Konjugat freigesetzt (Brechbiel, M.W. et al., Inorg. Chem. 25: 2772 [1986]) und stört damit die physikalische Messung und als Folge den diagnostischen Wert.

Bei den bisher klinisch verwendeten Verbindungen (z.B. MAG 3 [EP 0250013], ECD [EP 0279417] oder HM-PAO [EP 0123504]), bestimmt der Komplex, dessen ausschließliche Funktion es eigentlich sein soll via seiner $\gamma$-Strahlung die physikalische Messung zu ermöglichen auch die biologische Verteilung. Damit sind diese Komplexe zur Kopplung an niedermolekulare sich im Organismus selektiv anreichernde Molküle ungeeignet. Darüber hinaus enthalten diese Komplexbildner funktionelle Gruppen, die in unerwünschte Wechselwirkung mit dem lebenden Organismus treten können.

Es besteht daher ein dringlicher Bedarf an stabilen leicht zugänglichen Komplexen, die an unterschiedliche, sich per se im Organismus selektiv anreichernden Verbindungen gekoppelt sind und deren Anreicherungsverhalten ausschließlich durch diese niedermolekularen organischen Moleküle bestimmt wird.

Erfindungsgemäß wird diese Aufgabe durch Verbindungen der allgemeinen Formel I gelöst,

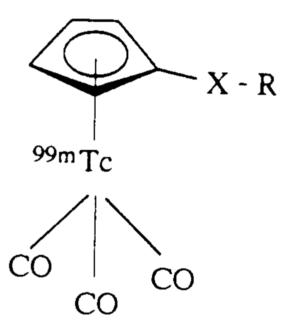

(I)

worin

X für eine Carbonylgruppe oder eine direkte Bindung,

R für einen Phenyl- oder Benzylrest,

oder für einen gesättigten oder ungesättigten, geradkettigen oder verzweigten

$C_{1-16}$-Kohlenwasserstoffrest, der gegebenenfalls ein bis drei Carbonylgruppen und/oder ein bis drei Carboxygruppen und/oder einen $C_{1-3}$ - Alkyl-, einen Phenyl - oder Benzylcarbonsäureester und/oder einen $NR_1R_2$ - Rest enthält, worin $R_1$ und $R_2$ gleich oder unterschiedlich sind und für Wasserstoff, Deuterium, einen geradkettigen oder verzweigten $C_{1-6}$ - Alkyl -, einen Desoxysaccharid - oder einen Desaminoergolin-Rest stehen,

oder für eine $-(CH_2)_lNR_3R_4$ - Gruppe, worin

l = 0 oder 1 und $R_3$, $R_4$ gleich oder unterschiedlich sind und für Wasserstoff, einen geradkettigen oder verzweigten, gesättigten oder ungesättigten $C_1$-$_6$ - Alkylrest - der gegebenenfalls durch einen $C_{1-3}$ - Alkyl-, Phenyl- oder Benzylcarbonsäureester oder einen Carbonsäurerest substituiert ist - stehen oder gemeinsam mit dem Stickstoff-Atom einen gesättigten oder ungesättigten 5 oder 6 Ring bilden, der gegebenenfalls ein Sauerstoffatom oder eine $NR_5$ - oder eine $CHR_5$-Gruppe mit $R_5$ in der Bedeutung eines Wasserstoffs, Deuteriums, geradkettigen oder verzweigten $C_{1-4}$ Alkylrestes enthält,

oder $NR_3R_4$ für einen 2-Nitroimidazol-Rest oder einen biogenen Amin-Rest steht,

oder für ein um das saure (Alkohol-)Proton vermindertes Alkoholmolekül steht, oder worin X-R für

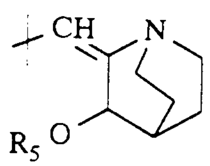
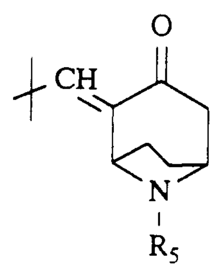
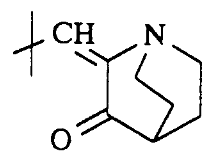

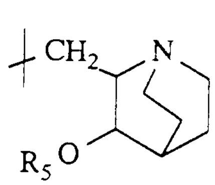
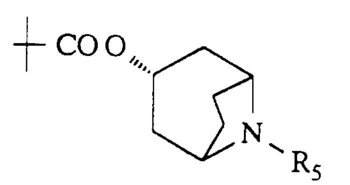
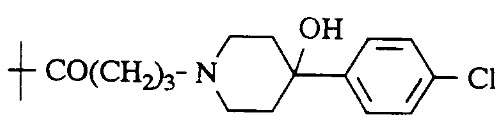

steht.

Erfindungsgemäß bevorzugt sind diejenigen Verbindungen gemäß Anspruch 1 worin -XR CO-CH=CH-$C_5H_6$ , $-CO-NH-CH_2COOH$ , $-CO-CH_2COOEt$ , $-CO-(CH_2)_2COOH$ , $-CO-(CH_2)_{14}COOH$ , $-CH=CH-CO-CH_3$ , $-CH_2-CH(NH^{iso}C_3H_7)CH_3$ , $-CH_2-CH(NH^{iso}C_3H_7)C_2H_5$ , -C≡CH , -CO-C≡CH , $-CO-(CH_2)_7COOH$, oder worin der Alkohol-Rest RO- ein $C_{1-6}$ - Alkyl-, Phenyl- oder Benzylalkohol-, ein cyclischer aliphatischer Aminoalkohol, Tropanol-, Chinuclidinol-, 3-Cholesterol-, 3-Estradiol- oder 17-Estradiol-Rest, oder worin der gesättigte oder ungesättigte Stickstoff enthaltende 5 oder 6 Ring Pyrrol, Pyrazol, Pyrazin, Pyridin, Morpholin, Pyrrolidin, Pyrimidin, Piperidin, Piperazin, 4-$R_5$-Piperidin, N-$R_5$-Piperazin, mit $R_5$ in der Bedeutung eines geradkettigen oder verzweigten $C_{1-4}$-Alkylrest bevorzugt für Methyl, Ethyl, Propyl, iso-Propyl steht, ist.

Als biogenes Amin seien beispielhaft genannt, Serotonin, Histamin, γ-Aminobuttersäure. Als Beispiel für einen cyclischen aliphatischen Aminoalkohol sei genannt

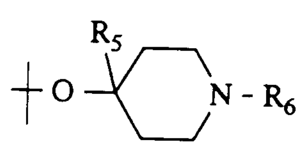

wobei $R_6$ für die für $R_5$ angegebenen Reste steht.

Überraschenderweise erfüllen viele der erfindungsgemäßen Cyclopentadienylcarbonyl-$^{99m}$Tc-Komplexe-im folgenden Cytectrene genannt - das gestellte Anforderungsprofil. So weisen die erfindungsgemäßen Komplexe gegenüber den in der Erprobung befindlichen Komplexen wie z.B. HM-PAO zur Himperfusion eine deutlich höhere Stabilität auf. So wird selbst von Metaboliten der applizierten Komplexe kein $^{99m}$Tc-Technetium freigesetzt.

*Biochemische Untersuchungen*

Als weiterer Vorteil der erfindungsgemäßen Komplexe, ist die Flexibilität der Organspezifität zu sehen. Von den Technetium-markierten Cytectren-Derivaten wurden die Organverteilungen bei Mäusen und Ratten sowie teilweise bei Kaninchen untersucht. Die Organverteilung der $^{99m}$Tc markierten Cytectren-Derivate ist abhängig von dem Substituenten am Cyclopentadienyl-Ringsystem.
Fig. 1 gibt die Organverteilung verschiedener Cytectren-Derivate an. Zum Vergleich wurde in dieser Tabelle auch die Organverteilung von Pertechnetat aufgenommen. Aus den Organ-Konzentrationen ist eindeutig zu erkennen, daß die Organverteilung der Cytectren-Derivate sich drastisch von der Verteilung nach Gabe von Pertechnetat abhebt. Beim Pertechnetat ist das Organ mit der höchsten Radioaktivitäts-Konzentration das Blut (2,6 % der Dosis/% Körpergewicht). Dagegen zeigen die $^{99m}$Tc Cytectren-Derivate eine ausgeprägte Affinität zu anderen, jeweils verschiedenen Organen. Die Affinität zu dem Einzelorgan ist eindeutig von der Seitenkette der Cytectren-Verbindung abhängig.
Beispiele für eine praktische Anwendung ergeben sich bei der Hippuran-analogen Cytectren-Verbindung zur Nieren-Diagnostik und den Estern mit Chinuclidinol oder 4-Hydroxy-(N-methyl)-piperidin zur Gehirn-Darstellung.
So ergaben Untersuchungen an der Ratte (siehe Fig. 2 und 3), daß die Konzentration im Gehirn z.B. für den erfindungsgemäßen Cyclopentadienylcarbonsäure-(N-methyl-piperidinol)-4-ester-$^{99m}$technetium-tricarbonyl-Komplex (hergestellt in Analogie zu Beispiel 6) ca. 4% Dosis/% Körpergewicht beträgt, während vergleichbare Messungen für bekannte Verbindungen, wie z.B. die erwähnten HM-PAO Komplexe, Werte um 1,7% Dosis/% Körpergewicht ergaben (L. Bacciottini et al., Europ. J. Nucl. Med., 17(1990)242. Außerdem ist das Konzentrationsverhältnis Gehirn/Blut etwa um den Faktor 10 besser.
Diese Ergebnisse konnten durch vergleichende Studien am Kaninchen nach Applikation von 14 MBq der genannten erfindungsgemäßen Verbindung, mit einer bezogen auf die Aktivität vergleichbaren Menge des HM-PAO Komplexes bestätigt werden (siehe experimenteller Teil) und kommen in einer deutlicheren visuellen Abgrenzbarkeit des Gehirns bei SPECT-Untersuchungen zum Ausdruck.

*Synthese*

Das erfindungsgemäße Verfahren eröffnet erstmals den Zugang zu den erfindungsgemäßen $^{99m}$Tc-markierten Radiodiagnostika vom Cytectren-Typ mit einer unbegrenzten Vielfalt für die Seitenkette -XR. Die Herstellung der erfindungsgemäßen Komplexe erfolgt indem man $^{99m}$Tc-Pertechnetat mit einer den Substituenten -XR tragenden neutralen oder positv geladenen Cyclopentadienyl-Verbindung des Eisens, Chroms oder Cobalts der allgemeinen Formel III oder IV

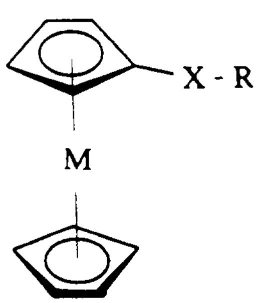

(III)

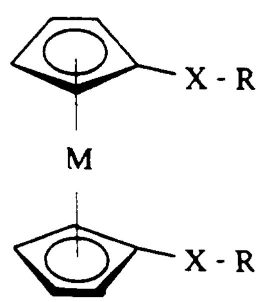

(IV)

worin M für Eisen, Chrom oder Cobalt steht und -XR die genannte Bedeutung hat, unter Anwesenheit eines Carbonylgruppendonators wie z.B. $Mn(CO)_5Br$, $Fe_2(CO)_9$ oder Natriumformiat, vorzugsweise $[FeCp(CO)_2]_2$ oder Oxalsäure, gegebenenfalls unter Zugabe eines Reduktionsmittels, wie z.B. Zinn(II)chlorid, Hydrazin, Dithionit oder Natriumthiosulfat, vorzugsweise in einem Lösungsmittel wie z.B Tetrahydrofuran, Methanol, Ethanol bzw. deren wässrige Mischungen, gegebenenfalls unter Zugabe eines Katalysators wie z.B. Thalliumcyclopentadienyl, Thalliumacetat, Magnesiumchlorid oder Cyclopentadienyltitandichlorid in einem geschlossenen Gefäß oder in der Schmelze bei Temperaturen zwischen 50 und 210 °C, vorzugsweise bei 120 - 170 °C umgesetzt wird.

Die Zugabe von Reduktionsmitteln ist bei den vorbekannten Verfahren in der Regel notwendig, um das als Generator-Eluat anfallende $^{99m}$Tc-Pertechnetat (Oxidationsstufe +7) in eine zum Einbau in den Komplex geeignete Oxidationsstufe (im Falle der erfindungsgemäßen Verbindungen +1) zu reduzieren. Überraschenderweise ist bei Ferrocenderivaten die Zugabe eines separaten Reduktionsmittels bei dem erfindungsgemäßen Verfahren häufig überflüssig, da das eingesetzte Metallocen selbst als Reduktionsmittel wirken kann.

Unter den Carbonyl-Donatoren sind diejenigen bevorzugt, die Mangan-frei sind, wie z.B $[FeCp(CO)_2]$ oder Oxalsäure.

Die Verwendung dieser Donatoren erlaubt die "trägerfreie" Herstellung der erfindungsgemäßen Cytectrene. Unter "trägerfrei" wird verstanden, daß der gewünschte $^{99m}$Tc-Technetium-Komplex nicht durch Komplexe mit gleichen Liganden aber anderen Zentralatomen verunreinigt ist. Diese oft unerwünschten analogen Mangan-Komplexe können bei der Verwendung von Mangan-haltigen Carbonyl-Donatoren in einer Nebenreaktion gebildet werden. Eine Abtrennung dieser Nebenprodukte z.B. mit Hilfe einer Kieselgelsäule gelingt im Falle des Mangans aufgrund ähnlicher $R_F$-Werte nicht, bzw. nur mit für die klinische Anwendung unakzeptablem Aufwand.

Diese Nebenprodukte treten bei der Anlagerung an Rezeptoren in Konkurrenz mit der radioaktiven Spezies bzw. verdrängen den $^{99m}$Tc-Technetium-Komplex von seiner Bindungsstelle.
Die Synthese und Abtrennung trägerfreier $^{99m}$Tc-Cytectren-Derivate stellt somit einen entscheidenen Vorteil gegenüber den üblichen - in der Klinik hergestellten - "trägerfreien"' $^{99m}$Tc-Komplexe dar. In den dafür verwendeten vorkonfektionierten Gläschen ist nämlich der Komplexbildner (Chelat) im Überschuß in einer Menge von 0,5 - 2 mg vorhanden, der nach $^{99m}$Tc-Pertechnetat-Zugabe einen trägerfreien Technetium-Komplex bildet.
Körpereigene Ionen können mit dem unverbrauchten Überschuß an Chelatbildnern ebenfalls einen analogen Komplex bilden, der ähnlich von dem zu untersuchenden Rezeptor gebunden wird wie der radioaktive Komplex. Das bedeutet: Im Körper selbst gebildete Ionen-Komplexe können zumindest teilweise den Technetium-Komplex von seiner Bindungsstelle verdrängen und damit zu einer verminderten $^{99m}$Tc-Anreicherung führen. Gerade diese Möglichkeit ist bei den nach dem erfindungsgemäßen Verfahren hergestellten $^{99m}$Tc-Cytectrenen ausgeschlossen.
Durch die Synthese "tragerfreier" $^{99m}$Technetiumkomplexe wird erreicht, daß an die Rezeptoren ausschließlich der gewünschte radioaktive Komplex gebunden wird, wodurch erstmals die Darstellung von z.B. Dopamin-Rezeptoren, die bislang lediglich über PET oder $^{123}$Jod-SPECT Aufnahmetechniken möglich war, möglich wird.

Wie Untersuchungen ergaben liegt die bevorzugte Reaktionstemperatur für diesen Zentralatomaustausch im Bereich zwischen 120 - 170 °C. Dies wird z.B. durch Fig. 4, die die radiochemische Ausbeute der nach Bespiel 1 hergestellten Verbindung zeigt, belegt. Diese Temperaturen können in der Klinik z.B. in einem Dampfdrucksterilisator erzielt werden. Die Reaktionszeiten liegen im Bereich von 15 bis 60 Minuten.

Eine Abtrennung der Technetium-markierten-Verbindung von radioaktiven Nebenprodukten und nicht-radioaktiven Begleitstoffen ist am einfachsten durch Dünnschicht-Chromatographie oder HPLC zu erreichen. Oft gelingt dies aber auch mit Hilfe einer Kleinen Kieselgel-säule, die mit dem Reaktionsansatz beladen und anschließend mit unterschiedlichen Eluationsmitteln eluiert wird.

Die Herstellung der erfindungsgemäßen pharmazeutischen Mittel erfolgt in an sich bekannter Weise, indem man die erfindungsgemäßen Cytectrene - gegebenenfalls unter Zugabe der in der Galenik üblichen Zusätze - in Wasser, Alkohol oder deren Gemischen löst und anschließend sterilfiltriert. Geeignete Zusätze sind beispielsweise physiologisch unbedenkliche Puffer (z.B. Tromethamin), geringe Zusätze von Elektrolyten (z.B. Natriumchlorid), Stabilisatoren (z.B. Gluconat, Phosphate oder Phosphonate).

Bei der nuclearmedizinischen In-vivo-Anwendung werden die erfindungsgemäßen Mittel in Mengen kleiner als $10^{-10}$ mol/Kg Körpergewicht dosiert. Ausgehend von einem mittleren Körpergewicht von 70 kg beträgt die Radioaktivitätsmenge für diagnostische Anwendungen zwischen 180 - 1100 MBq, vorzugsweise 500 - 800 MBq pro Applikation. Die Applikation wird normalerweise durch intravenöse, intraarterielle, peritoneale oder intratumorale Injektion von 0,1 bis 2 ml einer Lösung der erfindungsgemäßen Mittel erfolgen. Bevorzugt ist die intravenöse Applikation.

Die nachfolgenden Beispiele dienen der näheren Erläuterung des Erfindungsgegenstandes.

<u>Material und Methoden</u>

Die Ferrocen-Derivate waren zum kleineren Teil käufliche Produkte, zum größten Teil wurden sie entsprechend den Angaben der Literatur synthetisiert Das Tc-99m Pertechnetat war das übliche Eluat (mit physiol. NaCl-Lösung) eines Molybdan-Generators. Die radioaktiven Dünnschichtplatten wurden mit dem Berthold Dünnschicht-Scanner LB 2322 direkt gemessen.

Beispiel 1a)

**Tc-99m Acetyl-Cytectren aus Diacetyl-Ferrocen**

In eine Glasampulle gibt man 3 mg Diacetylferrocen, 6 mg $Mn(CO)_5Br$, 1 mg $MnCl_2$, 1,5 mg $Na_2S_2O_4$, 100 µCi $TcO_4^-$ (enthalten in 10 µl Eluat eines Tc-Generators) sowie 0,1 ml Methanol. Die abgeschmolzene Ampulle wird 1 h auf 100 °C. erhitzt.
Der Ampulleninhalt wird anschließend auf Kieselgel-DC-Platten der Fa. Merck aufgetragen und Cyclohexan/Essigester (7 : 3) chromatographiert. Wie ein Chromatogramm zeigt, erhält man bei $R_F$0,36 ein Maximum, das 16% der eingesetzten $^{99m}$Tc-Aktivität enthält.
Der $R_F$-Wert ist identisch mit dem $R_F$-Wert der dabei gebildeten Mn-haltigen Substanz, dem Acetyl-Cymantren, dessen Konstitution folgender Formel entspricht: $C_5H_4$-CO-$CH_2$ • Mn • $(CO)_3$.
Wird in obigem Ansatz das Reduktionsmittel Dithionit durch 0,1 mg $SnCl_2$ ersetzt, so steigt die Ausbeute an $^{99m}$Tc-Acetylcymantren auf über 60%.
Diese lipophile $^{99m}$Tc-Verbindung kann in reiner Form durch Eluation der entsprechenden Kieselgel-Fraktion ($R_F$ = 0,36) z.B. mit Ethanol gewonnen werden. Durch Rechromatographie des Eluates wird die radiochemische Reinheit bewiesen. Die markierte Verbindung ist chemisch stabil, denn nach 24h ist chromatographisch keine Zersetzung feststellbar.
Die Organ-Verteilung bei Mäusen zeigt eine bevorzugte Anreicherung in Leber und Nebenniere für $^{99m}$Tc-Acetyl-Cymantren und damit ein vollkommen verschiedenes Verhalten im Vergleich zum $^{99m}$Tc- Pertechnetat.

Beispiel 1b)

In eine Glasampulle gibt man
3 mg Diacetylferrocen,
6 mg $Mn(CO)_5Br$,
1 mg $MnCl_2$, 0,1 mg $SnCl_2$,
100 µCi $TcO_4^-$ (enthalten in 10µl Eluat eines Tc-Generators) sowie 0,1 ml Methanol.
Die abgeschmolzene Ampulle wird 1 h auf 130 °C. erhitzt.

Der Ampulleninhalt wird anschließend auf Kieselgel-DC-Platten der Fa. Merck aufgetragen und Cyclohexan/Essigester (7:3) chromatographiert. Auf dem Chromatogramm erhält man bei $R_F$ 0,36 ein Maximum, das 60 % der eingesetzten $^{99m}$Tc-Aktivität enthält.

Der $R_F$-Wert ist identisch mit dem $R_F$-Wert der dabei gebildeten Mn-haltigen Substanz, dem Acetyl-Cymentren, dessen Konstitution folgender Formel entspricht: $C_5H_4$-CO-$CH_3$ • Mn • $(CO)_3$(Nachweis durch Massenspektrum).

Diese lipophile $^{99m}$Tc-Verbindung kann in reiner Form durch Eluation der entsprechenden Kieselgel-Fraktion ($R_F$ = 0,36) z.B. mit Ethanol gewonnen werden. Durch Rechromatographie des Eluates wird die radiochemische Reinheit bewiesen. Die markierte Verbindung ist chemisch stabil, nach 24 h ist chroamtogrphisch keine Zersetzung feststellbar.

Beispiel 2

**Synthese: Tc-99m Cytectren-CO-Ergolin**

Die Ferrocenvorstufe Fc-CO-6-methylergolin wurde aus dem aktivierten Ferrocencarbonsäureester und 6-Methylergolin erhalten.
In einer Ampulle waren enthalten:
1,5 mg Fc-CO-6-methylergolin
4 mg $Mn(CO)_5Br$
1 mg Tl-acetat
in 0,2 ml THF 20 % $H_2O$
1h 170 °C.
Ausbeute: 64 %

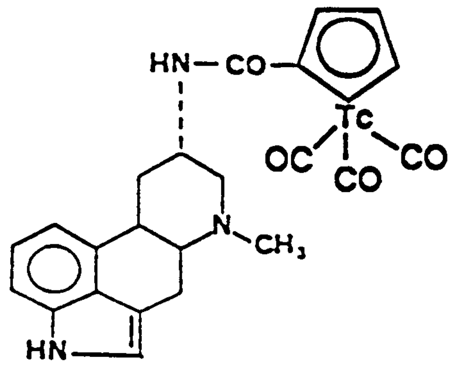

Das Dünnschichtchromatogramm des ungereinigten Syntheseansatzes ist Fig. 5, die der gereinigten $^{99m}$Technetium-markierten Verbindung den Figuren 6 bzw. 7, zu entnehmen.

Beispiel3

Tc-99m Cytectren-CO$(CH_2)_{14}$-COOH aus Ferrocen-CO-$(CH_2)_{14}$-COOH

In einer Glasampulle werden
2 mg Fc-CO-$(CH_2)_{14}$-COOH
4 mg $Mn(CO)_5Br$
0,2 ml Tetrahydrofüran mit 30 % Wasser
0,02 ml Pertechnetat-Lösung mit 11,7 MBq
1 h auf 150 ° C. erhitzt. Anschließend wird der Ampulleninhalt quantitativ auf ein Kieselgelplatte strichförmig aufgetragen und in Chloroform/Aceton/Ameisensäure (75 : 20 : 1,5) chromatographiert. Das Radioaktivitäts-Maximim mit dem $R_F$ 0,74 (Gelbfärbung durch das Ferrocen-Ausgangsprodukt) wird abgeschabt und diese Kieselgel-Fraktion mit Aceton eluiert.
Bei einer Rechromatographie im gleichen Laufmtttel und in Cyclohexan/Essigester (60:40) erwies sich die Tc-99m-Verbindung als einheitlich; hier war der RF-Wert 0,17 mit dem RF-Wert der Ferrocen-Fettsäure ebenfalls identisch.
Radiochem. Ausbeute: 63 %.

Beispiel 4

Tc-99m-Cytectren-"Haloperidol" aus Ferrocen-Haloperidol

a) Zur Herstellung der trägerfreien Cytectren-Verbindung (siehe Formel unten) werden in einer Glasampulle
2 mg Ferrocen-"Haloperidol"
4 mg $[Fecp(CO)_2]_2$
1 mg $MgCl_2 \cdot 6H_2O$
0,2 ml Tetrahydrofuran
0,02 ml Pertechnetat-Lösung mit 0,5 MBq
1 h auf 170 ° C. erhitzt. Anschließend DC in Ethanol/Aceton/Ammoniak (5 : 95 : 0,5), Fc-Haloperidol: $R_F$ = 0,60, Radiochem. Ausbeute an Cytectren-"Haloperidol" ($R_F$ = 0,71), 41%. Wählt man als Carbonyl-Donator 4 mg Oxalsäure, so liegt die radiochem. Ausbeute bei 21 %.
b) Zur Herstellung der Cytectren-Verbindung (im Gemisch mit Cymantren-"Haloperidol") gibt man in eine Glasampulle
2 mg Ferrocen-"Haloperidol"
4 mg $Mn(CO)_5Br$
0,2 ml Ethanol
0,02 ml Pertechnetat-Lösung mit 0,7 MBq
und erhitzt die zugeschmolzene Ampulle 1 h auf 150 ° C., Radiochem. Ausbeute: 93 %, mit THF als Lösungsmittel 95 %.
Das Tc-99m Cytectren-"Haloperidol" wird durch UV-Licht (z.B. beim längerem Betrachten von Dünnschichtchromatogrammen unter einer UV-Lampe) langsam zersetzt.

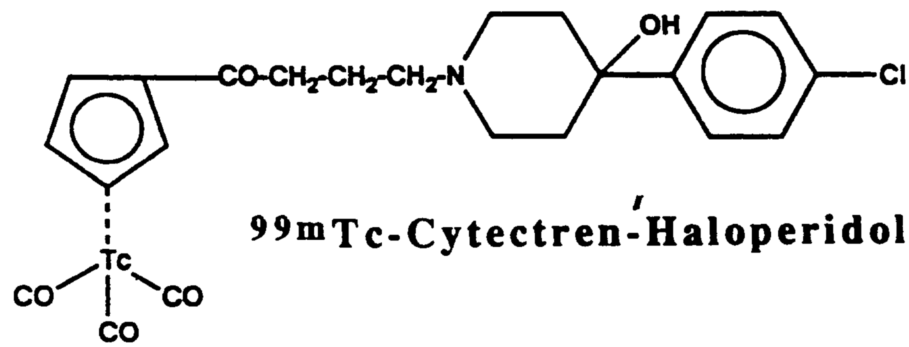

„$^{99m}$Tc-Cytectren-Haloperidol"

Beispiel 5

Ester der Tc-99m-Cytectrencarbonsäure mit Estradiol aus 3-Estradiol-Ferrocen-carboxylat

In einer Glasampulle werden
1 mg 3-Estradiol-Ferrocencarboxylat
2 mg ($Mn(CO)_5Br$
1 mg Tl-acetat
0,2 ml Tetrahydrofuran mit 30 % Wasser
0,02 ml Pertechnetat-Lösung mit 1,05 MBq
1 h auf 170 °C. erhitzt. DC in Cyclohexan/Essigester (60 : 40), Radiochem. Ausbeute des Cytectrenesters ($R_F$ = 0,33) 31%. Vgl. auch Chromatogramme Fig. 8 und 9.

Beispiel 6

Tc-99m 3-Chinuclidinol-Cytectren-carboxylat aus 3-Chinuclidinol-ferrocen-carboxylat

In eine Ampulle werden
2 mg Chinuclidinol-ferrocen-carboxylat
2 mg $Mn(CO)_5Br$
0,2 ml Tetrahydrofuran mit 30 % Wasser
0,02 ml Pertechnetat-Lösung mit 1,6 MBq
1 h auf 150 °C. erhitzt. Der Ampulleninhalt wird durch DC in Ether/Diethylamin (95 : 5) chromatographiert.
$R_F$ des Ferrocenesters und der Cytectrenester bei 0,25. Radiochem Ausbeute: 64 %
Eine Trennung der beiden Ester im Laufmittel Ethanol/Aceton/$NH_3$ (5 : 95 : 0,5) ist möglich.

Beispiel 7

Tc-99m-Benzoyl-cytectren aus Dibenzoyl-Ferrocen

Je Glasampulle (A und B) werden zugesetzt:
3 mg Dibenzoyl-Ferrocen
3 mg $Mn(CO)_5Br$
1 mg Cyclopentadienyl-Thallium
0,2 ml Tetrahydrofuran mit 30% Wasser
0,02 ml Pertechnetat-Lösung mit 5 MBq Tc-99m
Ampulle B enthielt zusätzlich 0,5 mg $SnCl_2$
Die abgeschmolzenen Ampullen werden 1 h auf 120 ° erhitzt und der Inhalt anschließend auf einer Kieselgel-Dünnschichtplatte in Cyclohexan/Essigester (7 : 3) chromatographiert. Dibenzoyl-Ferrocen: $R_F$ 0,33
Radiochem. Ausbeute an Benzoyl-Cytectren ($R_F$ = 0,44), Ampulle A: 19 %, Ampulle B: 36 % Literaturstellen für die Herstellung der eingesetzten Ausgangsverbindungen, sowie weitere beispielhaft ausgewählte erfindungsgemäße $^{99m}$Tc-Komplexe, sind der nachfolgenden Tabelle zu entnehmen.

Tabelle

| Ferrocen-Ausgangsprodukte | Temp. | Ausbeute | Lösungsmittel |
|---|---|---|---|
| Fc(-CO-Ph)$_2$ 1) | 120°C. | 92 % | MeOH |
| Fc(-CO-NH-CH$_2$-COOEt)$_2$ 1) (1) | 130°C. | 12 % | MeOH+3%HCl |
| Fc-CO-NH-CH$_2$-COOH (1) | 150°C. | 31 % | THF |
| Fc(-COOMe)$_2$ 1) | 150°C. | 25 % | MeOH+3%HCl |
| Fc(-COCH$_3$)$_2$ 1) | 140°C. | 98% | MeOH |
| Fc-CO-CH=CH-Ph (2) | 130°C. | 45 % | MeOH+3%HCl |
| Fc-COOCH$_3$ | 150°C. | 63 % | THF+ 30%H$_2$O |
| Fc-CONH$_2$ | 150°C. | 51 % | THF+ 30%H$_2$O |
| Fc-CO-CH$_2$-COOEt 2) | 150°C. | 61 % | THF+ 30%H$_2$O |
| Fc-CO-(CH$_2$)$_2$-COOH 2) | 150°C. | 90 % | THF |
| Fc-CO-(CH$_2$)$_7$-COOH 2) | 150°C. | 26 % | EtOH |
| Fc-CO-(CH$_2$)$_{14}$-COOH (3) | 150°C. | 69 % | THF+ 30%H$_2$O |
| Fc-(CH$_2$)$_{15}$-COOH (3) | 170°C. | 29 % | EtOH |
| Fc-Haloperidol (4) | 150°C. | 95 % | EtOH,THF+30%H$_2$O |
| N-(Fc-CH$_2$-)-Glukosamin (5) | 150°C. | 18 % | THF+ 30%H$_2$O |
| Fc-COO- 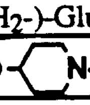 N-CH$_3$ ortho, meta | 150°C. | 58 % | THF+ 30%H |
| Fc-COO-3-Chinuclidinol (6) | 150°C. | 61 % | THF+ 30%H$_2$O |
| Fc-COO-3-Cholesterol (7) | 150°C. | 32 % | THF+ 30%H$_2$O |
| Fc-COO-3-Estradiol (7) | 170°C. | 31 % | THF+ 30%H$_2$O |
| Fc-COO-17-Estradiol (7) | 170°C. | 24 % | THF+ 30%H$_2$O |
| Fc-CH=CH-CO-CH$_3$ | 170°C. | 41 % | MeOH |
| Fc-CH$_2$-CH(-NH-isopropyl)-CH$_3$ (8) | 150°C. | 45 % | THF+ 30%H$_2$O |

Fortsetzung Tab.

| Ferrocen-Ausgangsprodukte | Temp. | Ausbeute | Lösungsmittel |
|---|---|---|---|
| Fc-$CH_2$-CH(-NH-isopropyl)-$C_2H_5$ (8) | 150°C. | 46 % | THF+ 30%$H_2O$ |
| Fc-C≡CH (10) | 170°C. | 4 % | THF |
| Fc-CO-C≡CH 2) | 150°C. | 49 % | EtOH |
| N-(Fc-CO-)-Ergolinamin | 150°C. | 65 % | EtOH |
| 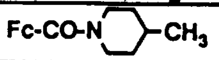 3) | 170°C. | 81 % | EtOH |
| 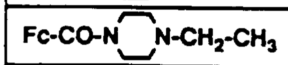 3) | 170°C. | 75 % | EtOH |
| 2-Ferrocenyliden-chinuclidin-3-on (9) | 150°C. | 40 % | EtOH |
| 2-Ferrocenyliden-chinuclidin-3-ol (9) | 150°C. | 31 % | THF |
| 2-Ferrocenyliden-tropin-3-on 4) | 150°C. | 12 % | MeOH |
| 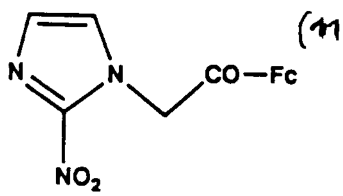 (11) | 150°C. | 29 % | EtOH |

Fc = Ferrocenyl-

Der Zentralatom-Austausch erfolgte ohne Zusatz von $SnCl_2$ und ohne Zusatz von Katalystoren, wie z.B. Tl-acetat oder $MgCl_2$ (siehe Beispiele).
Die Literaturstellen ( ) zur Tabelle sind in anhängender Zusammenstellung enthalten.

1) Das gebildete Cytectren-Derivat ist nur mono-substituiert.

2) Die Ferrocen-Ausgangsprodukte erhielt man durch Umsetzung von Ferrocen mit den entsprechenden Säurechloriden bzw. Anhydriden nach Fiedel-Craft.

3) Erhalten durch Umsetzen von Fc-COCl mit den entsprechenden Aminen.

4) Herstellung der Ferrocen-Verbindung analog zu (9).

Literatur zur Tab.

(1) Wenzel, M., Meinhold, H. und Schachschneider, G., Eur. J. Nucl. Med. *10*, 138 (1985).

(2) Dissertation Nipper, E., FU Berlin 1977.

(3) Privatmitteilung, I.H. Park (1988)

(4) Wenzel, M. und Wu, Y., Appl. Radiat. Isotopes *39*, 1237 (1988).

(5) Schneider, M. und Wenzel, M., J. Labelled Comp. and Radiopharmac. *18*, 293 (1981).

(6) Wenzel, M., J. Labelled Comp. Radiopharmac., *27*, 369 (1989).

(7) Hoffmann, K., Rießelmann, B. und Wenzel, M., Liebigs Ann. Chem. 1181 (1980).

(8) Wenzel, M., Langstaedtler, M. und Preiß, D., J. Appl. Radiat. Isot. *39*, 1023-1027 (1988).

(9) Wenzel, M., J. Labelled Comp. Radiopharmac. *28*, 1001 (1990).

(10) Sterzo, C. und Stille, J.K., Organometallics *9*, 687 (1990).

(11) N(Ferocenyl-methylen)-2-nitro-imidazol entsteht durch Reaktion von Bromessigsäure mit Nitroimidazol; anschließend wird die entstehende Carbonsäure in das Säurechlorid überführt, das mit Ferrocen nach Friedel-Craft zum gewünschten Produkt reagiert.

Beispiel einer In-vivo Studie:

In einer In-vivo Studie am Kaninchen (Neuseeländer, Züchtung Schering AG Berlin, ~4 kg, männlich) wurde die Gehirndurchblutungvon handelsüblichem HM-PAO mit dem N-Methylpiperidin-4-ol-cytectren-carbonsäureester (hergestellt analog Beispiel 6) verglichen.
Die Studie wurde mit einer SPECT-Kamera vom Typ Apex 409 ausgeführt.
Den Tieren wurde nach Nembutal-Narkose 1 ml einer isotonisch wässrigen, sterilen Lösung $^{99m}$Tc-HMPAO bzw. das genannte Cytectren-Derivat i.v. appliziert.
Die Aufnahmetechnik und die Ergebnisse wird durch die nachfolgende Zusammenstellung beschrieben.

| Aufnahmetechnik: | | |
|---|---|---|
| | **HM-PAO** | **Cytectren** |
| applizierte Aktivität: | 59 MBq $^{99m}$Tc-HM-PAO | 14 MBq $^{99m}$Tc-Cytectren |
| Untersuchungsdauer: | 2400 Sekunden | 2400 Sekunden |
| Untersuchungsbeginn: | 8 Minuten p.i. | 15 Minuten p.i. |
| Aufnahmesystem: | APEX 409/APC 3 | APEX 409/APC 3 |
| Aufnahmemodus: | Kollimator | Kollimator |
| Filterung: | step and shoot | step and shoot |
| Zoomfaktor: | Hanning HN 0-1-1 | Hanning HN 0-1-1 |
| Matrixgröße: | 2 | 2 |
| | 64 x 64 $pixel^2$ | 64 x 64 $pixel^2$ |

| Ergebnisse: | | |
|---|---|---|
| | **HM-PAO** | **Cytectren** |
| Gehirnuptake: | 5,9 % der applizierten | 7,5 % der applizierten |
| Kontrast [Gehirn/Background]: | Aktivität 0.98 | Aktivität 0.98 |
| Rauschen: | 2 %[($\sigma$ cts/pixel) ($\mu$cts/pixel)$^{-1}$ 100] | 1,4 %[($\sigma$ cts/pixel) ($\mu$cts/ pixel)$^{-1}$ 100] |

**Patentansprüche**

**1.** Cyclopentadienylcarbonyl-$^{99m}$Tc-Komplexe der allgemeinen Formel I

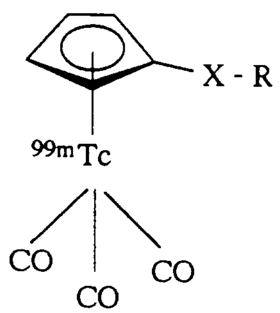

(I)

worin
X für eine Carbonylgruppe oder eine direkte Bindung,
R für einen Phenyl- oder Benzylrest,
oder für einen gesättigten oder ungesättigten, geradkettigen oder verzweigten $C_{1-16}$ - Kohlenwasserstoffrest, der gegebenenfalls ein bis drei Carbonylgruppen und/oder ein bis drei Carboxygruppen und/oder einen $C_{1-3}$ - Alkyl-, einen Phenyl - oder Benzylcarbonsäureester und/oder einen $NR_1R_2$ - Rest enthält, worin $R_1$ und $R_2$ gleich oder unterschiedlich sind und für Wasserstoff, Deuterium, einen geradkettigen oder verzweigten $C_{1-6}$ - Alkyl -, einen Desoxysaccharid - oder einen Desaminoergolin-Rest stehen,
oder für eine $-(CH_2)_lNR_3R_4$ - Gruppe, worin
1 = 0 oder 1 und $R_3$, $R_4$ gleich oder unterschiedlich sind und für Wasserstoff, einen geradkettigen oder verzweigten, gesättigten oder ungesättigten $C_{1-6}$ - Alkylrest - der gegebenenfalls durch einen $C_{1-3}$ - Alkyl-, Phenyl- oder Benzylcarbonsäureester oder einen Carbonsäurerest substituiert ist - stehen oder gemeinsam mit Stickstoff einen gesättigten oder ungesättigten 5 oder 6 Ring bilden, der gegebenenfalls ein Sauerstoffatom oder eine $NR_5$ - oder eine $CHR_5$-Gruppe, mit $R_5$ in der Bedeutung eines Wasserstoff- oder Deuterium-Atoms oder eines geradkettigen oder verzweigten $C_{1-4}$ Alkylrestes, enthält,

oder $NR_3R_4$ für einen 2-Nitroimidazol-Rest oder einen biogenen Amin-Rest steht, oder für ein um das saure (Alkohol-)Proton vermindertes Alkoholmolekül steht, oder worin X-R für

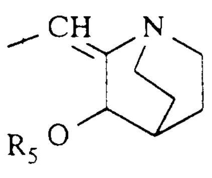

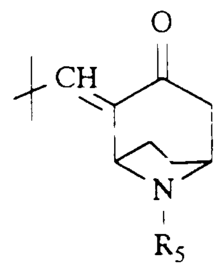

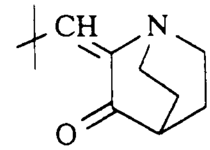

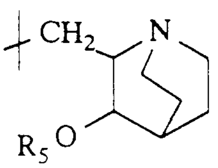

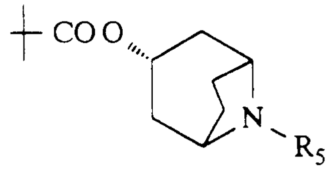

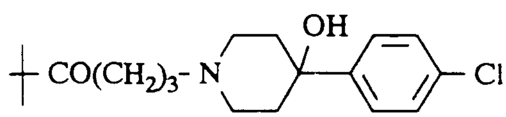

steht.

2. Verbindungen nach Anspruch 1, **dadurch gekennzeichnet**, daß der für R stehende Alkohol ein $C_{1-6}$ -Alkyl-, Phenyl- oder Benzylalkohol , ein cyclischer Aminalkohol oder ein Steroidalkohol ist.

3. Verbindungen nach Anspruch 2, **dadurch gekennzeichnet**, daß der cyclische Aminalkohol Tropanol, Chinuclidinol oder

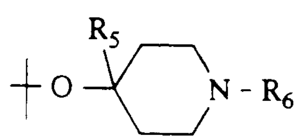

worin $R_6$ die unter $R_5$ angegebenen Reste steht, ist.

4. Verbindungen nach Anspruch 1, **dadurch gekennzeichnet**, daß das biogene Amin Serotin, Histamin oder $\gamma$-Aminobuttersäure ist.

5. Verbindungen nach Anspruch 2, **dadurch gekennzeichnet**, daß der Steroidalkohol 3-Cholesterol, 3-Estradiol oder 17-Estradiol ist.

6. Verbindungen nach Anspruch 1, **dadurch gekennzeichnet**, daß der für R stehende substituierte Kohlenwasserstoff-Rest ein Carboxy-$C_{2-16}$-Alkylen-Rest ist.

7. Verbindungen nach Anspruch 1, **dadurch gekennzeichnet**, das der für -XR stehende Rest $-CO-CH=CH-C_5H_6$ , $-CO-NH-CH_2COOH$ , $-CO-CH_2COOEt$ , $-CO-(CH_2)_2COOH$ , $-CO-(CH_2)_{14}COOH$ , $-CH=CH-CO-CH_3$ , $-CH_2-$,$CH(NH^{iso}C_3H_7)CH_3$ , $CH(NH^{iso}C_3H_7)C_2H_5$, $-C{\equiv}CH$, $-CO-C{\equiv}CH$, $-CO-(CH_2)_7COOH$ ist.

8. Verfahren zur Herstellung von Verbindungen der allgemeinen Formel 1, **dadurch gekennzeichnet**, daß $^{99m}$Pertechnetat mit einer den Substituenten -XR tragenden Cyclopentadienyl-Verbindung des Eisens, Chroms oder Cobalts unter Anwesenheit eines Carbonylgruppendonators wie z.B. $Fe_2(CO)_9$, $[FeCp(CO)_2]_2$, Oxalsäure oder Natriumformiat, vorzugsweise $Mn(CO)_5Br$, gegebenenfalls unter Zugabe eines Reduktionsmittels, wie z.B. Zinn(II)chlorid, Hydrazin, Dithionit oder Natriumthiosulfat, vorzugsweise in einem Lösungsmittel - welches gegebenenfalls mit z.B. Salzsäure angesäuert sein kann - wie z.B Tetrahydrofuran, Methanol, Ethanol, bzw. deren wässrige Mischungen, gegebenenfalls unter Zugabe eines Katalysators wie z.B. Thalliumcyclopentadienyl, Magnesiumchlorid, Cyclopentadienyltitandichlorid oder Thalliumacetat in einem geschlossenen Gefäß oder in der Schmelze bei Temperaturen zwischen

50 und 210 °C, vorzugsweise bei 120 - 170 °C umgesetzt wird.

**9.** Cyclopentadienylcarbonyl-$^{99m}$Tc-Komplexe gemäß Anspruch 1 zur Anwendung in Diagnostizierverfahren.

**10.** Diagnostische Mittel enthaltend mindestens einen Cyclopentadienylcarbonyl-$^{99m}$Tc-Komplex nach Anspruch I und galenische Hilfsstoffe.

**11.** Verfahren zur Herstellung eines diagnostischen Mittels nach Anspruch 10, dadurch gekennzeichnet, daß man die in Wasser oder Wasser-Alkohol-Gemischen gelösten Komplexe, gegebenenfalls mit in der Galenik üblichen Zusätzen, in eine für die Applikation geeignete Form bringt.

**12.** Diagnostische Mittel nach Anspruch 10, **dadurch gekennzeichnet**, daß die eingesetzte Radioaktivität im Bereich von 180 - 1100 MBq, vorzugsweise zwischen 500 - 800 MBq $^{99m}$Tc liegt.

**13.** Ein Kit zur Herstellung von Cyclopentadienylcarbonyl-$^{99m}$Tc-Komplexen gemäß Anspruch I, bestehend aus einem mit einem Septum verschlossenen Reaktionsgefäß, das einen den Substituenten XR tragenden Cyclopentadienyl-Komplex des Eisens, Chroms oder Cobalts, einen Carbonylgruppendonator und gegebenenfalls ein Reduktionsmittel sowie gegebenenfalls einen Katalysator in steriler Form enthält, in welches eine Pertechnetat-Lösung mittels einer Kanüle eingeführt und anschließend erhitzt werden kann.

## Claims

**1.** $^{99m}$Tc cyclopentadienylcarbonyl complexes of the general formula I

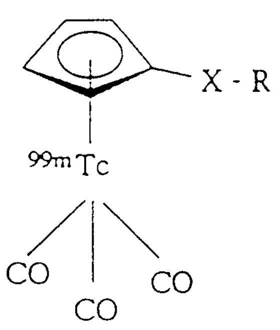

(I)

wherein

X is a carbonyl group or a direct bond,

R is a phenyl or benzyl radical or
a saturated or unsaturated, straight-chain or branched $C_{1-16}$hydrocarbon radical optionally containing from one to three carbonyl groups and/or from one to three carboxy groups and/or a $C_{1-3}$alkyl-, a phenyl- or a benzyl-carboxylic acid ester radical and/or an $NR_1R_2$ radical wherein $R_1$ and $R_2$ are identical or different and are hydrogen, deuterium, a straight-chain or branched $C_{1-6}$alkyl, a deoxysaccharide or a deaminoergoline radical, or
a $-(CH_2)_lNR_3R_4$ group wherein
l = 0 or 1 and $R_3$ and $R_4$ are identical or different and are hydrogen, a straight-chain or branched, saturated or unsaturated $C_{1-6}$alkyl radical - which is optionally substituted by a $C_{1-3}$alkyl-, phenyl- or benzyl-carboxylic acid ester radical or by a carboxylic acid radical - or together with nitrogen form a saturated or unsaturated 5- or 6-membered ring which optionally contains an oxygen atom or an $NR_5$ or $CHR_5$ group, with $R_5$ being a hydrogen or deuterium atom or being a straight-chain or branched $C_{1-4}$alkyl radical, or $NR_3R_4$ is a 2-nitroimidazole radical or a biogenic amine radical, or
an alcohol molecule missing the acid (alcohol)-proton, or wherein

X-R is

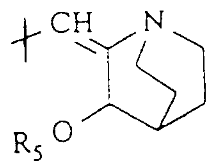
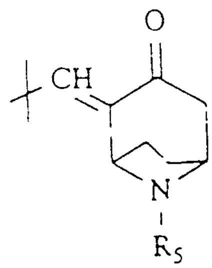
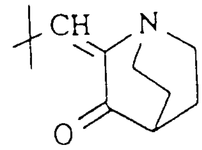
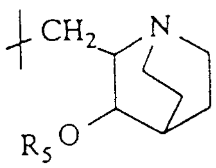
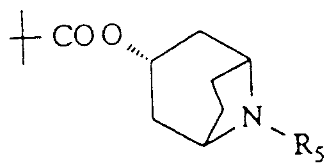
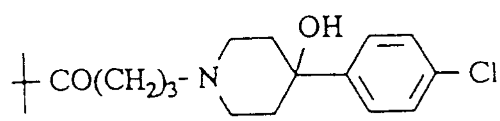

2. Compounds according to claim 1, **characterised in that** the alcohol represented by R is a $C_{1-6}$alkyl, phenyl or benzyl alcohol, a cyclic amino alcohol or a steroid alcohol.

3. Compounds according to claim 2, **characterised in that** the cyclic amino alcohol is tropanol, quinuclidinol or

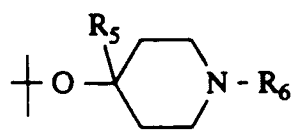

wherein $R_6$ represents the radicals given under $R_5$.

4. Compounds according to claim 1**, characterised in that** the biogenic amine is serotonin, histamine or γ-aminobutyric acid.

5. Compounds according to claim 2, **characterised in that** the steroid alcohol is 3-cholesterol, 3-oestradiol or 17-oestradiol.

6. Compounds according to claim 1, **characterised in that** the substituted hydrocarbon radical represented by R is a carboxy-$C_{2-16}$alkylene radical.

7. Compounds according to claim 1, **characterised in that** the radical represented by -XR is -CO-CH=CH-$C_5H_6$, -CO-NH-$CH_2$COOH, -CO-$CH_2$COOEt, -CO-$(CH_2)_2$COOH, -CO-$(CH_2)_{14}$COOH, -CH=CH-CO-$CH_3$, -$CH_2$-, CH(NH$^{iso}C_3H_7$)$CH_3$, CH(NH$^{iso}C_3H_7$)$C_2H_5$, -C≡CH, -CO-C≡CH or -CO-$(CH_2)_7$COOH.

8. A process for the preparation of compounds of the general formula I, **characterised in that** $^{99m}$pertechnetate is reacted with a cyclopentadienyl compound of iron, chromium or cobalt that carries the substituent -XR, in the presence of a carbonyl group donor such as, for example, $Fe_2(CO)_9$, [FeCp-$(CO)_2]_2$, oxalic acid or sodium formate, preferably $Mn(CO)_5Br$, optionally with the addition of a reducing agent, such as, for example, tin(II) chloride, hydrazine, dithionite or sodium thiosulphate, preferably in a solvent - which can optionally have been acidified with, for example, hydrochloric acid, - such as, for example, tetrahydrofuran, methanol, ethanol, or aqueous mixtures thereof, optionally with the addition of a catalyst such as, for example, thallium cyclopentadienyl, magnesium chloride, cyclopentadienyltitanium dichloride or thallium acetate in a closed vessel or in the melt at temperatures of from 50 to 210 °C, preferably at from 120 to 170 °C.

9. $^{99m}$Tc cyclopentadienylcarbonyl complexes according to claim 1 for use in diagnostic processes.

**10.** Diagnostic preparations comprising at least one $^{99m}Tc$ cyclopentadienylcarbonyl complex according to claim 1 and galenic excipients.

**11.** A process for the preparation of a diagnostic preparation according to claim 10, **characterised in that** the complexes, dissolved in water or water/alcohol mixtures, optionally with additives customary in galenics, are brought into a form suitable for application.

**12.** Diagnostic preparations according to claim 10, **characterised in that** the radioactivity used lies in the range of from 180 to 1100 MBq, preferably from 500 to 800 MBq, of $^{99m}Tc$.

**13.** A kit for the preparation of $^{99m}Tc$ cyclopentadienylcarbonyl complexes according to claim 1, consisting of a reaction vessel which is closed with a septum and which contains a cyclopentadienyl complex of iron, chromium or cobalt that carries the XR substituent, a carbonyl group donor and optionally a reducing agent and optionally a catalyst in sterile form, into which vessel a pertechnetate solution can be introduced by means of a cannula and then heated.

**Revendications**

**1.** Complexes de cyclopentadiènylcarbonyle-$^{99m}Tc$ de formule générale (I)

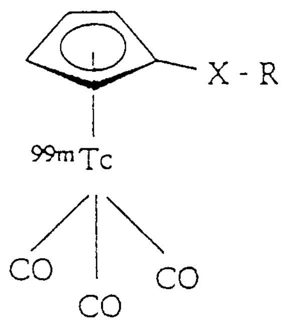

(I)

où

X est un groupe carbonyle ou une liaison directe,

R est un radical phényle ou benzyle, ou encore un radical hydrocarboné en $C_{1-16}$, saturé ou insaturé, à chaîne droite ou ramifiée, qui contient éventuellement de 1 à 3 groupes carbonyle et/ou de 1 à 3 groupes carboxy et/ou un ester d'un acide (alkyle en $C_{1-3}$)-, phényl- ou benzylcarboxylique et/ou un résidu $NR_1R_2$, où $R_1$ et $R_2$ sont identiques ou différents et représentent chacun un hydrogène, un deuterium, un radical alkyle en $C_{1-6}$ à chaîne droite ou ramifiée, un radical désoxysaccharide ou un radical désaminoergoline,

ou encore un groupe $-(CH_2)_lNR_3R_4$, où l = 0 ou 1, et $R_3$ et $R_4$ sont identiques ou différents et représentent chacun un hydrogène, un radical alkyle en $C_{1-6}$ à chaîne droite ou ramifiée, saturé ou insaturé, qui éventuellement est substitué par un ester d'un acide (alkyle en $C_{1-3}$)-, phényl- ou benzylcarboxylique ou par un résidu acide carboxylique, ou encore, avec l'atome d'azote, forment un noyau saturé ou insaturé à 5 ou 6 chaînons, lequel contient éventuellement un oxygène ou encore un groupe $NR_5$ ou un groupe $CHR_5$ où $R_5$ représente un hydrogène, un deuterium, ou encore un radical alkyle en $C_{1-4}$à chaîne droite ou ramifiée, ou bien $NR_3R_4$ est un radical 2-nitroimidazole ou un radical amine biogène,

ou encore une molécule d'un alcool diminué du proton acide (alcool),

ou encore où X-R est :

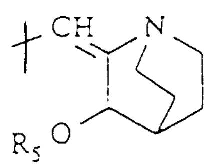
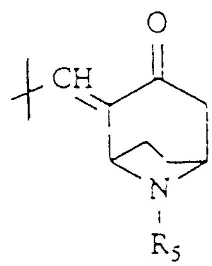
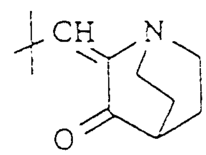
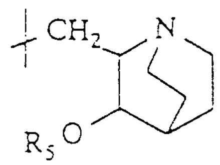
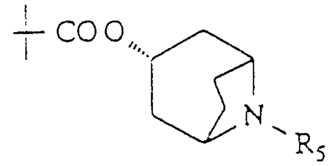
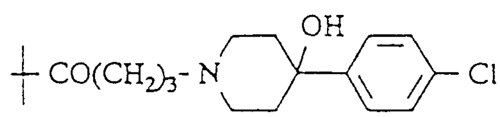

2. Composés selon la revendication 1, caractérisés en ce que l'alcool correspondant à R est un alcool alkylique en $C_{1-6}$, phénylique ou benzylique, un amino-alcool cyclique ou un alcool stéroïde.

3. Composés selon la revendication 2, caractérisés en ce que l'amino-alcool cyclique est le propanol, le quinuclidinol ou

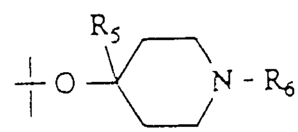

$R_6$ représente les radicaux mentionnés pour $R_5$.

4. Composés selon la revendication 1, caractérisés en ce que l'amine biogène est la sérotine, l'histamine ou l'acide $\gamma$-aminobutyrique.

5. Composés selon la revendication 2, caractérisés en ce que l'alcool stéroïde est le 3-cholestérol, le 3-oestradiol ou le 17-oestradiol.

6. Composés selon la revendication 1, caractérisés en ce que le radical hydrocarboné substitué représentant R est un radical carboxy-(alkylène en $C_{2-16}$).

7. Composés selon la revendication 1, caractérisés en ce que le radical correspondant à -XR est CO-CH=CH-$C_5H_6$,-CO-NH-$CH_2COOH$, CO-$CH_2COOEt$, -CO-$(CH_2)_2COOH$, -CO-$(CH_2)_{14}COOH$, -CH=CH-CO-$CH_3$, -$CH_2$-CH($NH^{iso}C_3H_7$)$CH_3$, -$CH_2$-CH($NH^{iso}C_3H_7$)$C_2H_5$,-C≡CH, -CO-C≡CH, -CO-$(CH_2)_7COOH$,.

8. Procédé pour préparer des composés de formule générale (I), caractérisé en ce qu'on fait réagir du $^{99m}$pertéchnétate avec un composé de cyclopentadiènyle portant le substituant -XR, du fer, du chrome ou du cobalt, en présence d'un donneur de groupes carbonyle tel par exemple que $Fe_2(CO)_9$, [FeCp-$(CO)_2$]$_2$, l'acide oxalique ou le formiate de sodium, de préférence le $Mn(CO)_5Br$, éventuellement avec addition d'un réducteur, tel par exemple que le chlorure d'étain(II), l'hydrazine, un dithionite ou le thiosulfate de sodium, de préférence dans un solvant, qui peut être éventuellement acidifié par exemple avec l'acide chlorhydrique, tel par exemple que le tétrahydrofuranne, le méthanol, l'éthanol ou leurs mélanges aqueux, éventuellement avec addition d'un catalyseur tel que le cyclopentadiènylthallium, le chlorure de magnésium, le dichlorure de cyclopentadiènyle ou l'acétate de thallium dans un récipient fermé ou à l'état fondu à des températures comprises entre 50 et 210 °C et de préférence entre 120 et 170 °C.

**9.** Complexes de cyclopentadiènylcarbonyle-$^{99m}$Tc selon la revendication 1, pour une utilisation dans des procédures de diagnostic.

**10.** Produits diagnostiques contenant au moins un complexe de cyclopentadiènylcarbonyle-$^{99m}$Tc selon la revendication 1 et des adjuvants galéniques.

**11.** Procédé pour préparer un produit diagnostique selon la revendication 10, caractérisé en ce qu'on donne une forme appropriée à l'administration aux complexes, dissous dans l'eau ou dans un mélange d'eau et d'un alcool, éventuellement avec des additifs usuels en galénique.

**12.** Produits diagnostiques selon la revendication 10, caractérisés en ce que la radioactivité utilisée est comprise entre 180 et 1100 et de préférence entre 500 et 800 MBq de $^{99m}$Tc.

**13.** Coffret pour préparer des complexes de cyclopentadiènylcarbonyle-$^{99m}$Tc selon la revendication 1, comportant un réacteur fermé par un septum, lequel contient un complexe de cyclopentadiènyle portant le substituant XR, du fer, du chrome ou du cobalt, un donneur de groupes carbonyle et éventuellement un réducteur, et éventuellement un catalyseur, dans lequel on peut introduire une solution de pertéchnétate à l'aide d'une canule, puis la chauffer.

Fig. 1

| Cytectren-Derivate mit Seitenkette X - R | Min. | Maus Ratte | Akt. Konzentration [% der inj. Dosis/% des Körpergewichts] | | | | | | | | Gehirn / Blut |
|---|---|---|---|---|---|---|---|---|---|---|---|
| | | | Muskel | Blut | Leber | Lunge | Niere | Nb.Niere | Herz | Gehirn | |
| Pertechnetat (Kontrolle) | 15 | Ms | 0,44 | 2,66 | 1,55 | 1,95 | 1,22 | 1,63 | 0,86 | 0,09 | 0,03 |
| | | | | | | | | | | | |
| 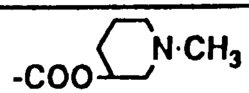 | 15 | R | 0,45 | 0,36 | 4,98 | 5,14 | 5,15 | - | 0,79 | 1,96 | 5,4 |
| 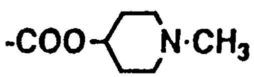 | 15 | R | 0,61 | 0,16 | 4,48 | 6,55 | 4,47 | - | 0,89 | 2,66 | 16,6 |
| -COO-3-Chinuclidinol | 15 | R | 0,64 | 0,32 | 4,19 | 13,8 | 7,01 | 8,64 | 1,44 | 2,29 | 7,2 |
| -CO-NH-$CH_2$-COOH | 15 | M | 0,21 | 0,40 | 2,27 | 0,37 | 24,9 | 0,67 | 1,30 | 0,06 | |
| -$CH_2$-CH($NH_2$)-$CH_3$ | 15 | Ms | 0,60 | 0,34 | 4,80 | 10,7 | 3,31 | 4,27 | 1,47 | 1,86 | 5,5 |
| -CO-$CH_3$ | 15 | Ms | 0,30 | 0,22 | 8,53 | 3,95 | 4,7 | 8,20 | 0,19 | 0,10 | |
| -CO-$(CH_2)_{14}$-COOH | 15 | Ms | 0,16 | 0,33 | 13,8 | 1,52 | 6,01 | 0,66 | 0,78 | 0,03 | - |
| -CH=Tropinon | 7,5 | Rt | 0,64 | 0,28 | 3,40 | 8,94 | 4,19 | 5,34 | 1,54 | 2,63 | 9,4 |

Fig. 2

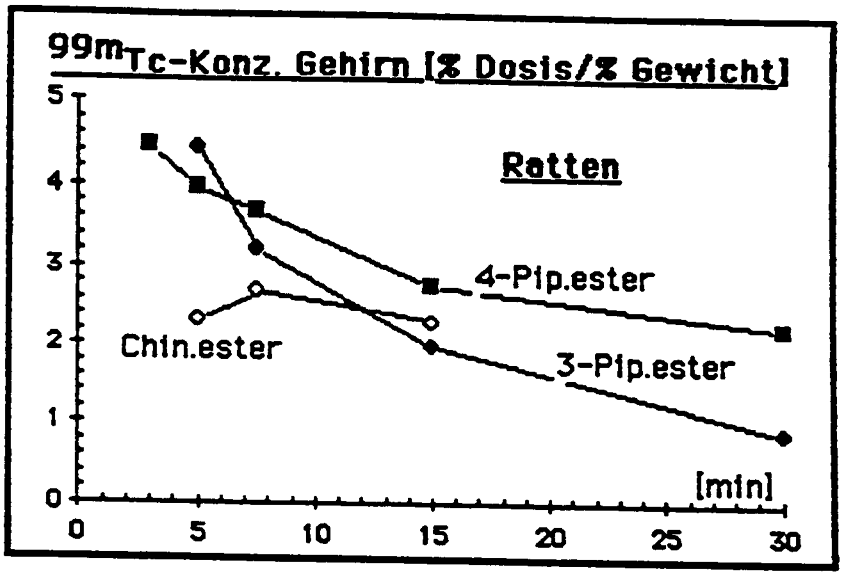


Die Ratten (n = 4) erhielten je Tier ca. 5 µCi der Tc-99 markierten Ester gelöst in 0,1 ml EtOH/0,9 % NaCl i.v.

Ester aus Cytectren-carbonsäure mit

| | |
|---|---|
| 4-Hydroxy-N-(methyl)-piperidin | = 4-Pip-ester |
| 3- " " " | = 3-Pip-ester |
| 3-Chinuclidinol | = Chin.-ester |

Die Gehirn-Konzentration 15 Minuten nach HM-PAO-Gabe liegt bei 1,7 % Dosis/% Gewicht.

Fig. 3

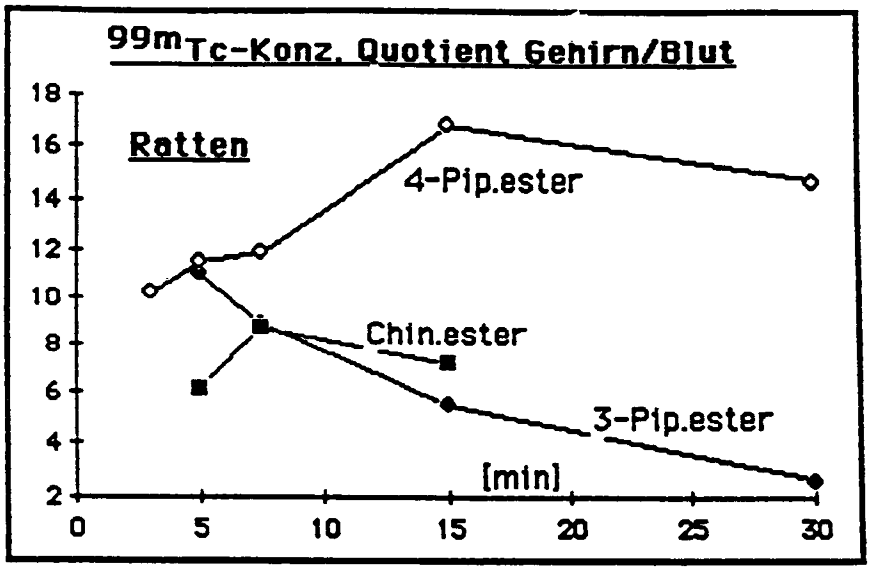


Die Ratten (n = 4) erhielten je Tier ca. 5 µCi der Tc-99 markierten Ester gelöst in 0,1 ml EtOH/0,9 % NaCl i.v.

Ester aus Cytectren-carbonsäure mit

| | |
|---|---|
| 4-Hydroxy-N-(methyl)-piperidin | = 4-Pip-ester |
| 3- " " " | = 3-Pip-ester |
| 3-Chinuclidinol | = Chin.-ester |

Der Gehirn/Blut Quotient 15 Minuten nach HM-PAO-Gabe liegt bei 1,5 : 1.

Fig. 4 Radiochemische Ausbeute bei der Synthese von $^{99m}$Tc-Acetyl-Cytectren
Reaktionszeit: 60 Minuten

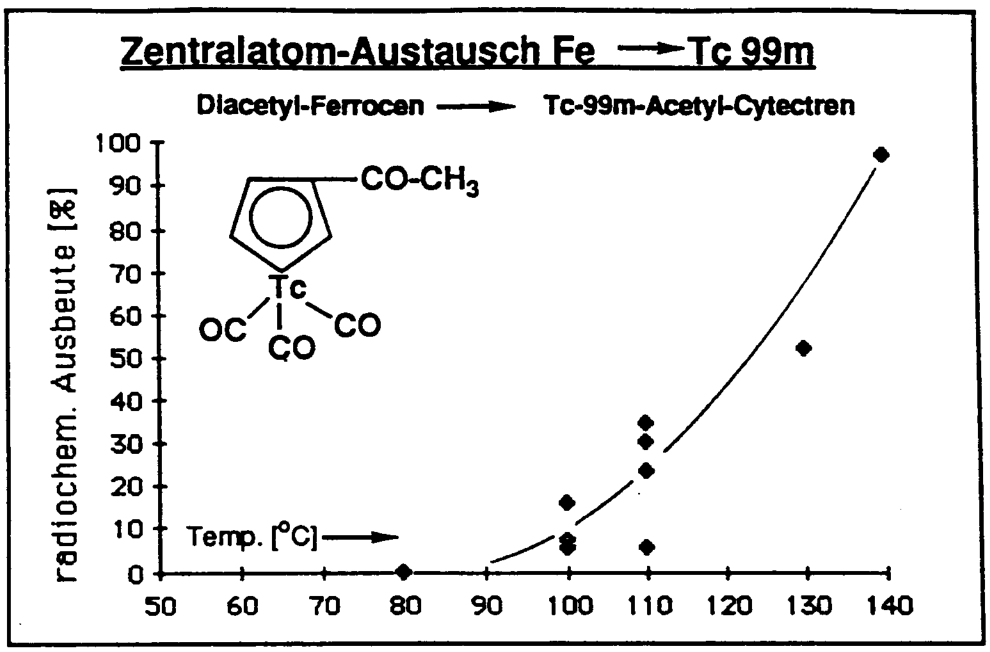

Fig. 5

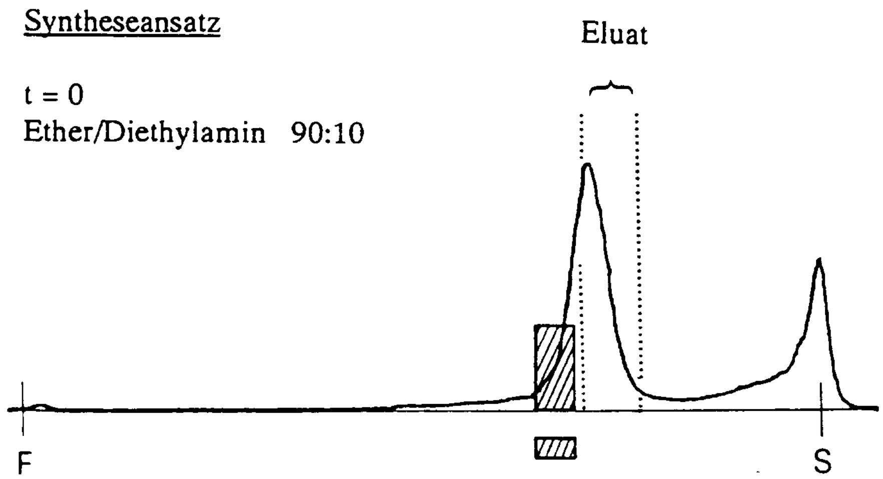

Fig. 6

Eluat

t = 24 h
Ether/Diethylamin 90:10

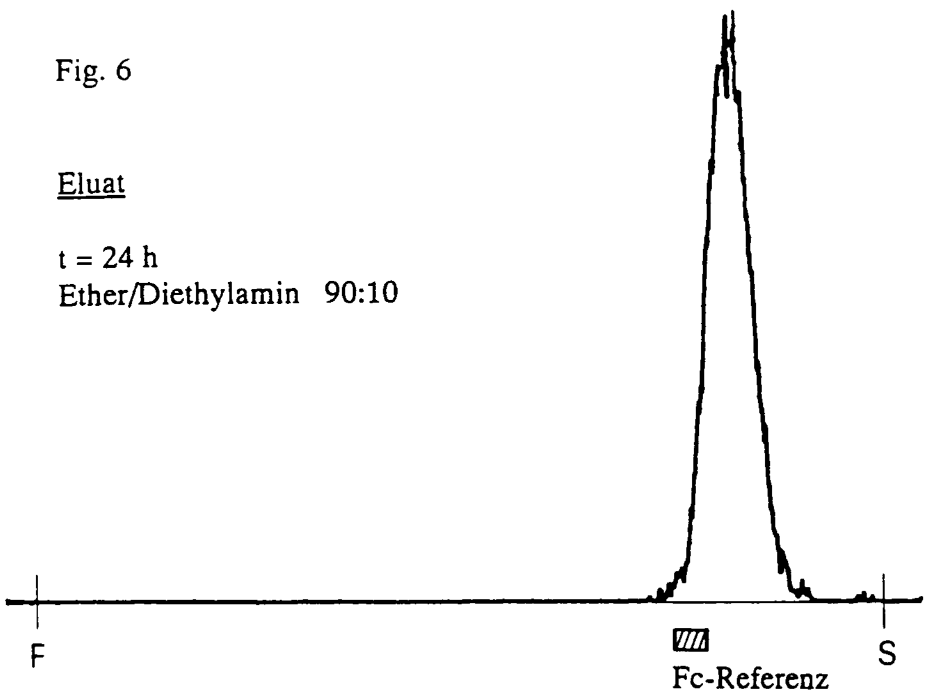


Fig.7

Eluat

t = 48 h
Aceton/EtOH/$NH_3$ 95:4:1

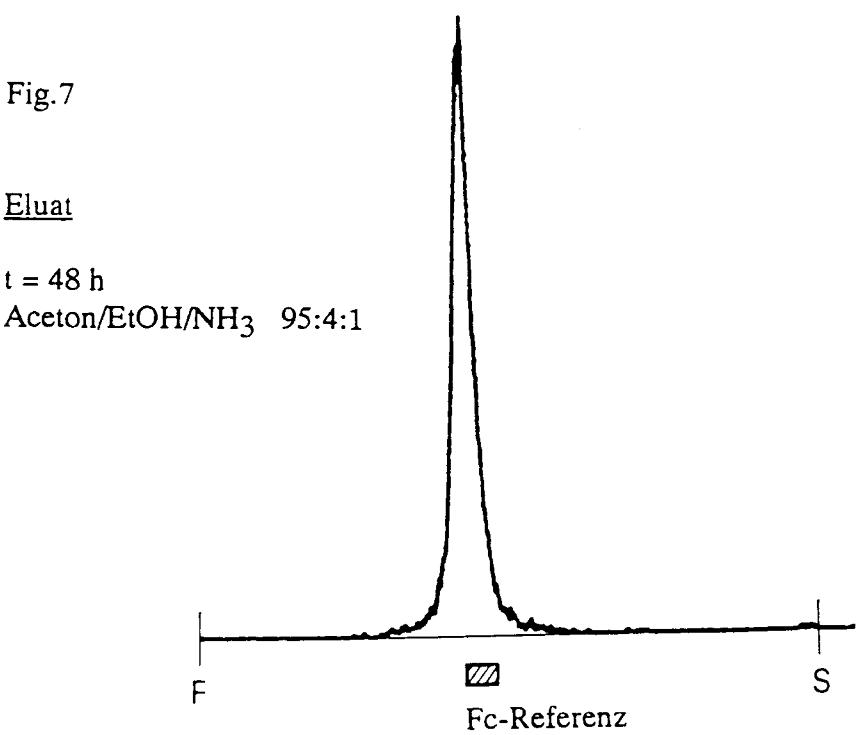

Fig. 8

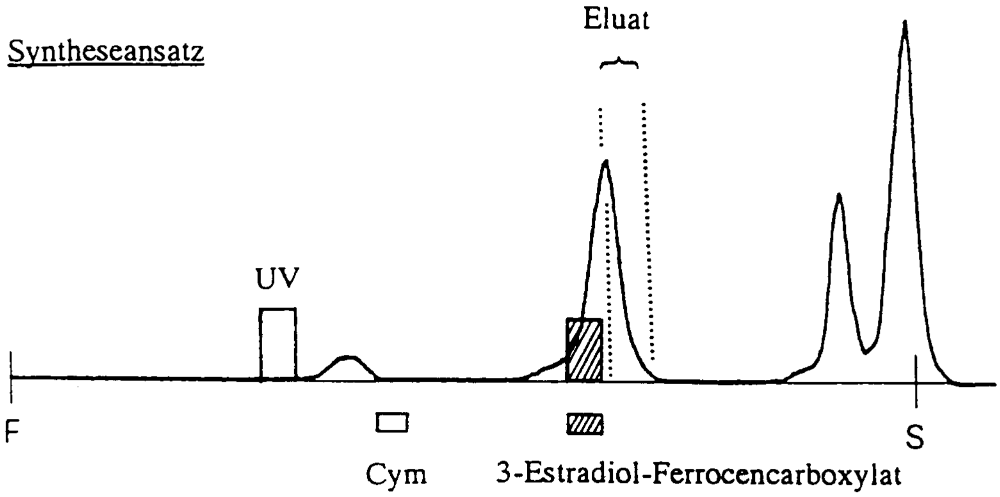
Syntheseansatz
Eluat
UV
F
S
Cym
3-Estradiol-Ferrocencarboxylat

Fig. 9

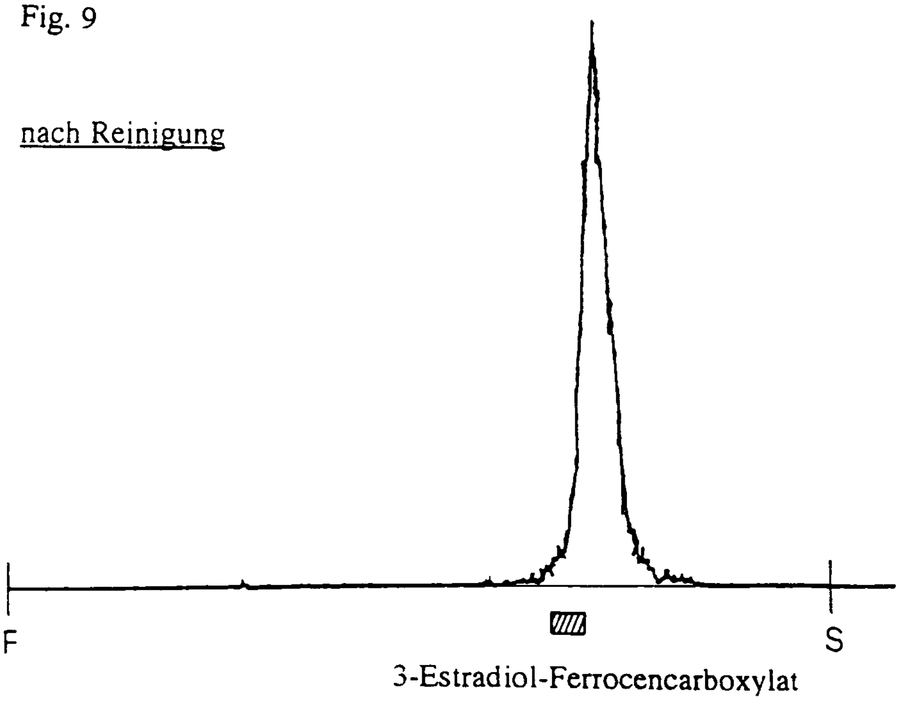
nach Reinigung
F
S
3-Estradiol-Ferrocencarboxylat